# EUROPEAN PATENT APPLICATION

(11) **EP 3 502 093 A1**
(43) Date of publication of application: **26.06.2019**
(21) Application number: 17208990.6
(22) Date of filing: 20.12.2017
(51) Int. Cl.: C07C 305/04, C07F 9/54, C07C 229/12

(54) **ZWITTERIONIC PILLARARENE DERIVATIVE**

(71) Applicant: Freie Universität Berlin, 14195 Berlin (DE)
(72) Inventor: HAAG, Rainer, 12209 Berlin (DE); GAO, Lingyan, 14197 Berlin (DE); LI, Mingjun, 14195 Berlin (DE)
(74) Representative: Maikowski & Ninnemann Patentanwälte Partnerschaft mbB

(57) **Abstract**

The present invention relates to a zwitterionic pillararene derivative having a structure according to the following general formula:

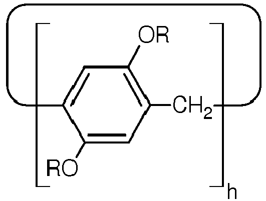

Thereby, residue R is a zwitterionic hydrocarbon chain. The invention further relates to uses of such zwitterionic pillararene derivative, to products comprising such zwitterionic pillararene derivative, and to a method for manufacturing such zwitterionic pillararene derivative.

## Description

The present invention relates to a zwitterionic pillararene derivative according to the preamble of claim 1, to a nanoparticle comprising such zwitterionic pillararene derivative according to the preamble of claim 6, to in vitro and in vivo uses according to the preambles of claims 7 and 9 as well as to a method for manufacturing such a zwitterionic pillararene derivative according to the preamble of claim 15.

In recent decades, bacterial infectious diseases are considered as a serious issue due to the rapid emergence, development and spread of antibiotic-resistant genes against frontline antimicrobials arising from the abuse/misuse and rational use of antibiotics in global healthcare and agriculture.¹ The increasing rates of antibiotic resistance and slow approval of drugs are compromising our ability to treat bacterial infections and cause a global health crisis.²

Besides the threat of antibiotic-resistant bacteria, biofilm-associated infections, which account for over 80 percent of microbial infections in the body, make the situation worse in combating pathogenic agents. The less effective agents remain, the more difficult it becomes to treat biofilm-enclosed bacteria. The resulting biofilms are able to cause severe infections in the bloodstream, leading to increased patient morbidity and medical expenses.³

Antimicrobial agents based on small molecules developed so far have shown strong ability to combat planktonic bacteria and prevent biofilm formation.^{4a, 4b} However, currently there are limited works about small molecules that can efficiently and specifically deal with pre-existing biofilms.^{5a, 5b} Therefore, one of the big challenges in industry, agriculture and clinical medicine is how to effectively treat bacterial infections and biofilm-associated diseases.

Antibiotics isolated from nature or prepared through chemical synthesis, quaternary ammonium compounds and other antibacterial agents have been widely used to combat the bacterial infections problem.⁶ However, in light of the drug resistance crisis, the pace of antibiotic discovery is still far too slow. Currently, antimicrobial peptides,⁶ cationic polymers,⁷ nanoparticles⁸ and photothermal/photodynamic agents^{9a, 9b} have been designed and used to replace conventional antibiotics to decrease the possibility of antibiotic resistance. Considerable efforts have been made with respect to the development of antibacterial nanomaterials as complementary to antibiotics due to their high surface area to volume ratio, and their unique physical and chemical properties.¹⁰

Supramolecular architectures, built upon the self-assembly of molecules *via* reversible noncovalent intermolecular forces, have been extensively studied as functional nanomaterials to explore versatile applications in different areas.¹¹ Macrocyclic compounds, as an important branch in supramolecular chemistry, have been widely employed to fabricate functional nanomaterials with specific properties and functions to realize different purposes.¹²

Pillararenes, as a burgeoning macrocycle species, have attracted much attention recently due to their particular pillar-shaped architectures and potentials in the preparation of functional assemblies used in different areas.^{13a, 13b} However, the self-assemblies of pillararenes have rarely been used in controlling the biological function of chemical molecules, especially in the field of combating bacteria.

Recently, Cohen and co-workers developed cationic pillar[5]arenes with ammonium or phosphonium groups that can inhibit biofilm formation of Gram-positive bacteria without affecting bacteria growth and viability.^{14a, 14b}

Hou and co-workers designed a pillar[5]arene with peptides as a transmembrane channel that can insert into the lipid bilayer of Gram-positive bacteria to show antimicrobial activity.^{15a, 15b}

The existing pillar[5]arenes designed for antibacterial purpose still have some challenges in facing planktonic bacteria and biofilm-matrix bacteria.

It is an object of the present invention to provide novel compounds that exhibit antimicrobial activity and that are suited to be used against bacteria, especially against bacterial biofilms.

This object is achieved by a zwitterionic pillararene derivative having the features explained in claim 1. Such zwitterionic pillararene derivative has a structure according to general formula (I):

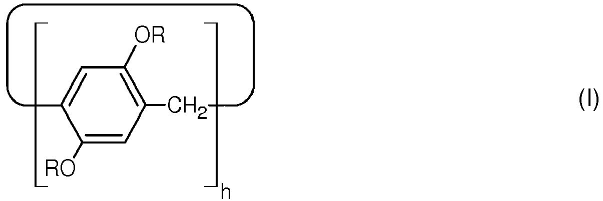

Thereby,
- R: is
- D: is OSO₃⁻ Y⁺, SO₃⁻ Y⁺, or CO₂⁻ Y⁺,
- E: is CH₃ or CH₂-CH₃,
- X: is Cl or Br,
- Y: is Na or K,
- Z: is N or P,
- h: is 5, 6, 7, 8, 9, or 10,
- m: is 1, 2, 3, 4, 5, 6, 7, 8, or 9, and
- n: is 1, 2, 3, 4, 5, 6, 7, 8, or 9.

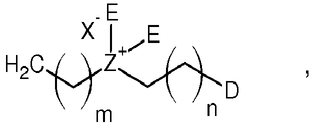

This zwitterionic pillar[h]arene demonstrated broad-spectrum antibacterial activity against Gram-negative bacteria, Gram-positive bacteria and drug-resistant bacteria. Besides, it exhibited excellent antiseptic ability against young and mature biofilms. In addition, after transferring the zwitterionic pillar[h]arene to a filter paper (zone of inhibition test), it showed durable and highly antibacterial effects against bacterial colonies.

Considering that pillararenes known from prior art were only active against Gram-positive bacteria, it was very surprising that the zwitterionic pillararenes described herein were also active against Gram-negative bacteria and drug-resistant bacteria. Additionally, the zwitterionic pillararenes exhibited their anti-bacterial activity already at very low concentrations. They were able to destroy pre-existing biofilms and bacterial colonies. These findings are particularly surprising since zwitterionic compounds are usually known to be bioinert. Thus, the inventors could not expect the achieved result.

The new class of molecules is structurally distinct from the pillararene structures reported in literature ^{14a, 14b, 15a, 15b}. Specifically, they distinguish themselves by a functionalized zwitterionic group, whereas pillararenes known from prior art are only cationic. This different charge (and charge distribution) of the presently claimed zwitterionic pillararenes might contribute to their superior antibacterial effects.

In an embodiment, the residue R corresponds to any of the following structures:

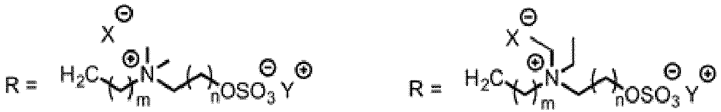

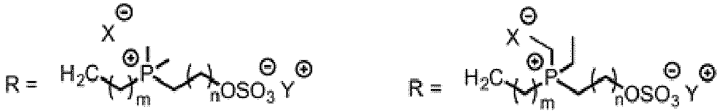

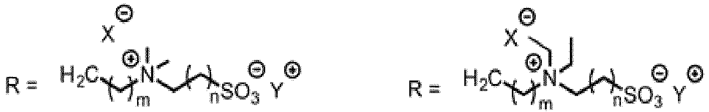

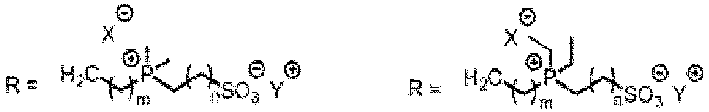

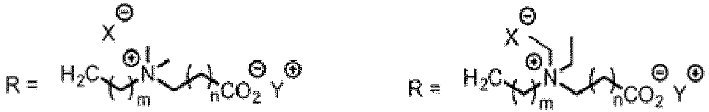

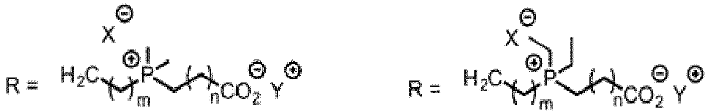

In an embodiment, h is 5 or 6. In such a case, the zwitterionic pillararene derivative is a pillar[5]arene or a pillar[6]arene, respectively. A pillar[5]arene is particular appropriate.

In an embodiment, E is CH₂-CH₃. Then, the residue Z (i.e., the nitrogen or phosphor atom) is substituted by two ethylene residues.

In an embodiment, m is 1 and n is 5. Then, the residue R of the zwitterionic pillararene derivative corresponds to the following structure:

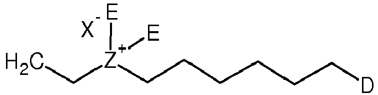

In an embodiment, D is OSO₃⁻ Y⁺. In such a case, the zwitterionic pillararene derivative is a sulfonate.

A particular appropriate zwitterionic pillararene derivative comprises a residue R corresponding to the following structure:

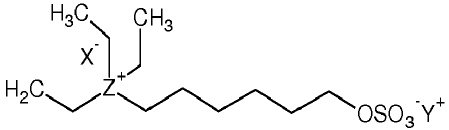

Another particular appropriate zwitterionic pillararene derivative comprises a residue R corresponding to the following structure:

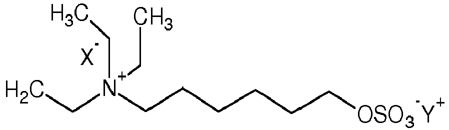

Another particular appropriate zwitterionic pillararene derivative comprises a residue R corresponding to the following structure:

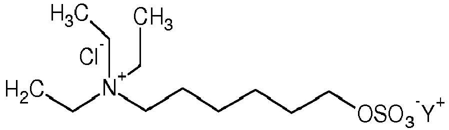

Another particular appropriate zwitterionic pillararene derivative comprises a residue R corresponding to the following structure:

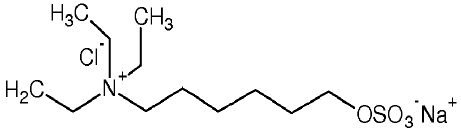

All of the before-mentioned residues R are, in an embodiment, part of a pillar[5]arene or a pillar[6]arene, in particular of a pillar[5]arene.

In an aspect, the present invention also relates to a nanoparticle comprising a plurality of zwitterionic pillararene derivative molecules according to the preceding explanations. Such a nanoparticle has a micelle-like structure. It turned out that the zwitterionic pillararene derivatives self-assemble (or aggregate) into such nanoparticles.

In an aspect, the present invention also relates to the use of a zwitterionic pillararene derivative or of a nanoparticle according to the preceding explanations as anti-biofilm compound and/or as antibacterial compound. This use particularly refers to an in vitro use.

In an embodiment, the anti-biofilm compound is used for providing a substrate with biofilm-inhibiting properties. Such a substrate can be coated or otherwise provided with the zwitterionic pillararene derivative or the nanoparticle in an industrial process. The substrate can then be stored until it shall be used. Generally, the substrate can be used for different purposes, such as in vitro or in vivo purposes.

In an aspect, the invention relates to method for preparing an anti-biofilm compound and/or an antibacterial compound by using a zwitterionic pillararene derivative or a nanoparticle according to the preceding explanations as active ingredient for promoting the anti-biofilm properties and/or antibacterial properties of the substrate. Thereby, the term "anti-biofilm" relates to an activity against an established biofilm, i.e., a pre-existing or pre-fabricated biofilm. An established biofilm is, in an embodiment, a structure having a defined architecture and comprising microbial cells and extracellular polymeric substances (EPS). An established biofilm typically provides an appropriate environment for an exchange of genetic material between microbial cells in the biofilm.

In an aspect, the present invention relates to the first medical use of a zwitterionic pillararene derivative or a nanoparticle according to the preceding explanations as medicament.

In an embodiment, the medicament is an anti-biofilm medicament and/or an antibacterial medicament.

In an aspect, the present invention relates to a method for treating a human or an animal in need thereof with a compound comprising a zwitterionic pillararene derivative or a nanoparticle according to the preceding explanations to prevent, inhibit and/or ameliorate a bacterial infection, to prevent the formation of a biofilm, to inhibit and/or ameliorate the growth of an existing biofilm, and/or to destroy and/or reduce an already formed biofilm.

In an embodiment, the animal is a mammal, in particular a rodent.

In an aspect, the invention also relates to an article (an item) comprising a matrix. This matrix comprises at least two constituents, namely a matrix material on the one hand and a zwitterionic pillararene derivative or a nanoparticle according to the preceding explanations on the other hand. Due to the presence of the zwitterionic pillararene derivative or the nanoparticle in or on the matrix, the matrix and therewith the whole article exhibits the properties provided by the zwitterionic pillararene derivative or the nanoparticle.

In an embodiment, the zwitterionic pillararene derivative or the nanoparticle is embedded into the matrix material. Thereby, a certain portion of the embedded zwitterionic pillararene derivative or the embedded nanoparticle is present on the surface of the matrix material and can thus interact with substances getting into contact with the surface of the matrix material.

In an embodiment, the zwitterionic pillararene derivative or the nanoparticle is additionally or alternatively coated onto a surface of the matrix material. Then, the concentration of the zwitterionic pillararene derivative or the nanoparticle on the surface of the matrix material can be adjusted as needed. In particular, higher surface concentrations of zwitterionic pillararene derivative or nanoparticle can often be achieved by coating than by embedding.

In an embodiment, the article is intended to be in contact with a human or animal body or a human or animal body fluid for a period of time lasting at least 6 hours, in particular at least 12 hours, in particular at least 24 hours, in particular at least 2 days, in particular at least 3 days, in particular at least 5 days, in particular at least 7 days, in particular at least 10 days, in particular at least 14 days, in particular at least 1 month, in particular at least 2 months, in particular at least 6 months, in particular at least 1 year, in particular at least 2 years, in particular at least 5 years, in particular at least 10 years. In an embodiment, the first period of time last between 6 hours and 10 years or any other time interval that can be built up from the before-mentioned lower limits of the period of time (e.g., 12 hours to 5 years, 7 days to 2 years etc.).

In an embodiment, the article is a stent, a prosthesis, an implant, a hose, a catheter, a bag or a medical device used in connection to infuse a substance to a human or an animal or to withdraw the body fluid from a human or an animal. Such medical device can be, in an embodiment, a port, a needle, a syringe or parts and fittings thereof.

In an aspect, the present invention relates to a method for manufacturing a zwitterionic pillararene derivative according to the preceding explanations. Such a zwitterionic pillar[h]arene can be prepared by a one-step reaction based on a basic pillararene framework. The manufacturing method comprises the following step:
reacting a compound of general formula (II) 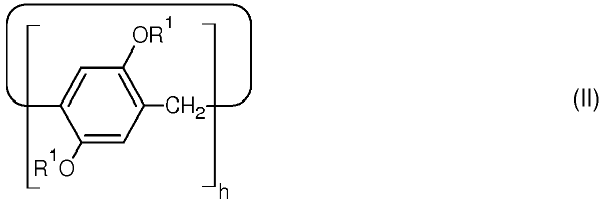
wherein
- R¹: is
- E: is CH₃ or CH₂-CH₃,
- Z: is N or P,
- h: is 5, 6, 7, 8, 9, or 10, and
- m: is 1, 2, 3, 4, 5, 6, 7, 8, or 9, 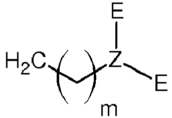
with a compound of general formula (III) 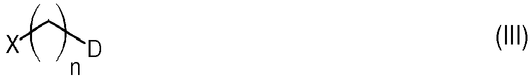 wherein
- D: is OSO₃⁻ Y⁺, SO₃⁻ Y⁺, or CO₂⁻ Y⁺,
- X: is Cl or Br,
- Y: is Na or K, and
- n: is 1, 2, 3, 4, 5, 6, 7, 8, or 9,
to obtain a zwitterionic pillararene derivative having a structure according to general formula (I) explained above.

In an embodiment, the reaction is carried out during a reaction period of several days (in particular 1 to 6 days, in particular 2 to 5 days, in particular 3 to 4 days). In an embodiment, the reaction is carried out at room temperature. Organic solvents such as dimethyl formamide (DMF) are particular appropriate solvents.

For example, zwitterionic pillar[5]arene can be easily manufactured by mixing 1 equivalent of the pillar[5]arene derivative (M1) and 20 equivalents of sodium 6-chlorohexyl sulfate (M4) in DMF. After a reaction period of several days (in particular 1 to 6 days, in particular 2 to 5 days, in particular 3 to 4 days) at 90 °C, the zwitterionic pillar[5]arene (ZW-PA) results. This reaction is shown in the following sheme:

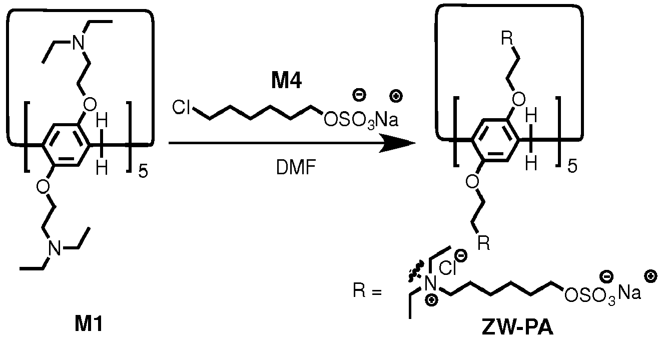

All embodiments can be combined in any desired way. Embodiments of the described zwitterionic pillararene derivative can be transferred in any desired combination to the described nanoparticle, to the described medical and uses non-medical uses and methods as well as to the described article, and in each case vice versa.

Details of aspects of the invention will be described in the following with respect to exemplary embodiments and accompanying Figures. In the Figures:
- Figure 1: shows the antibacterial ability of ZW-PA against *E.coli* (plot (a)), *S*. *aureus* (plot (b)) and kanamycin-resistant *E*. *coli* (plot (c));
- Figure 2: shows the behavior of *E*. *coli* biofilms grown for 1 day, 3 days or 5 days in response to a treatment with PBS, ampicillin and ZW-PA nanoparticles for 24 h; and
- Figure 3: shows the behavior of *E*. *coli* biofilms grown for 1 day in response to a treatment with PBS, ampicillin and ZW-PA nanoparticles for 24 h, 72 h or 120 h;

A pillar[5]arene corresponding to the following structure was manufactured as zwitterionic pillararene derivative: 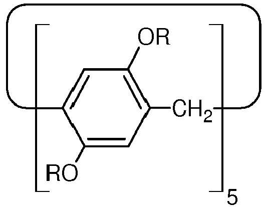 with R being

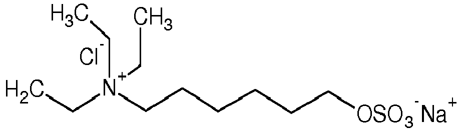

It will be referred to in the following as zwitterionic pillar[5]arene (ZW-PA).

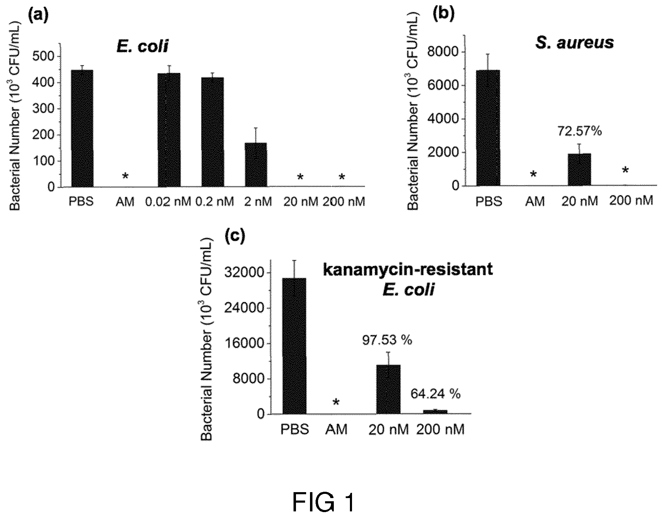

As depicted in Figure 1, this ZW-PA exhibited in solution remarkable antibacterial behavior against *Escherichia coli* (*E. coli*) with more than 99.99% efficiency at an extremely low concentration of 20 nM (Figure 1, plot (a)). For *Streptococcus aureus* (*S. aureus*) (Figure 1, plot (b)) and drug-resistant bacteria (kanamycin-resistant *E. coli*) (Figure 1, plot (b)), the ZW-PA solution demonstrated excellent antibacterial activity at a concentration of 200 nM. Ampicillin (AM, 20 nM) was used as positive control and phosphate-buffered saline (PBS) as negative control. The error bars in Figure 1 indicate the standard deviations of triplicate experiments. The percentages denote the efficiency in inhibiting bacterial growth.

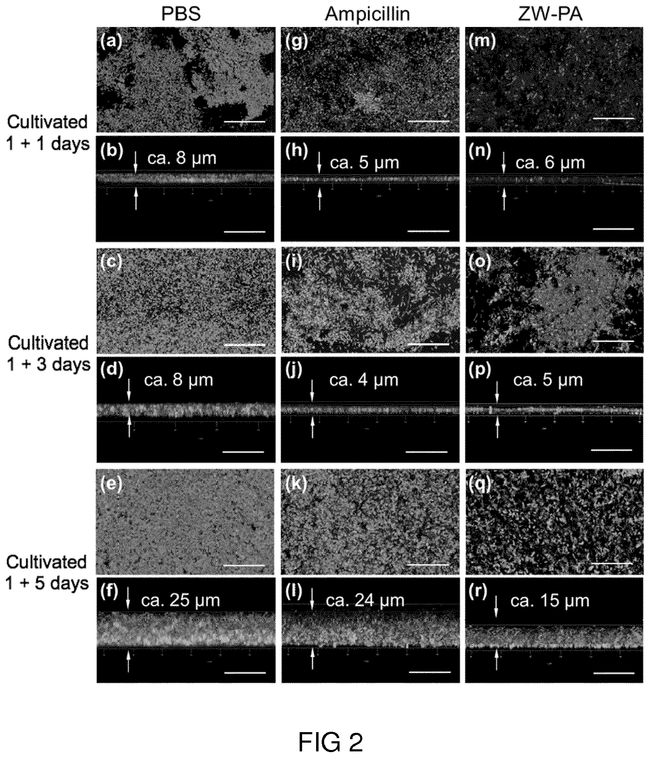

As depicted in Figure 2, *E. coli* biofilm in different stages - namely, in colony formation stage (1 day growth ["cultivated 1 + 1 days"]) and in maturation stages (3 days growth ["cultivated 1 + 3 days"] or 5 days growth ["cultivated 1 + 5 days"], respectively), were pre-fabricated according to literature¹⁶ and then treated with a dose of 200 nM ZW-PA nanoparticles, 200 nM ampicillin or PBS for another 24 h. Afterwards, the resulting biofilms on the 2^{nd} day, the 4^{th} day and the 6^{th} day were observed. A LIVE/DEAD staining of the *E*. *coli* cells was applied. By this staining, living cells are stained green, while dead cells are stained red. The staining revealed that the density and height of biofilm without treatment by ampicillin or ZW-PA nanoparticles gradually increased when the cultivation time increased from 1 +1 days to 1 + 3 days or 1 + 5 days. This is shown in Figure 2, plots (a)-(f). Specifically, the biofilm height increased from 8 µm on the 2^{nd} day (1^{st} + 1 day) to 25 µm on the 6^{th} day (1^{st} + 5 days). Only green (living) cells could be observed. The scale bars in all plots of Figure 2 represent 50 µm.

The results of pre-fabricated biofilms treated with ampicillin are depicted in Figure 2, plots (g)-(I). Compared with the control sample (Figure 2a and b), it could be observed that ampicillin at a concentration of 200 nM could not efficiently kill the cells in the young (1-day old) biofilm, which only exhibited loose cell density and relatively low height of 5 µm (Figure 2, plots (g) and (h)). However, already a significant number of cells, but less than the half of the cells, were stained red (dead cells).

The anti-biofilm ability of ampicillin became even worse when it was added to a biofilm cultivated for longer time. As shown in Figure 2, plot (i), although the cell density was not as high as the control shown in Figure 2, plot (c), most of the cells were still alive in the resulting biofilm of 4^{th} day (1^{st} + 3 days) in the presence of ampicillin for 24 h. In fact, the majority of the cells were stained green (living cells), only a considerable small amount of cells was stained red (dead cells).

For the 5-day grown biofilm, it could be observed on the 6^{th} day (1^{st} + 5 days) that ampicillin was effective only against the cells on the surface layer of the biofilm. It could not reduce the cell density of the biofilm, neither decrease the height of the biofilm that remained at 24 µm. Most cells were stained green (living cells). These results are depicted in Figure 2, plots (k)-(I). They suggest that the conventional antibiotic ampicillin could not efficiently penetrate and accumulate into dense biofilm matrix to combat a pre-existing biofilm. This finding is consistent with the published literature.¹⁶ For this reason, it was already described in literature to use antibiotic-loaded nanocarriers to achieve a better biofilm penetration.¹⁷

The images of Figure 2, plots (m)-(r) represent the resulting biofilms of different stages after incubation with ZW-PA nanoparticles for 24 h. There are significant differences between the biofilm samples treated with ampicillin or with ZW-PA nanoparticles. For the 2^{nd} day (1^{st} + 1 day), it could be observed that almost all the cells in the biofilm matrix were killed by ZW-PA; almost all cells were stained red (dead cells). This directly decreased the cell density of the biofilm. Besides, from the side view shown in Figure 2, plot (n), almost all the cells from top to bottom in the biofilm were killed by ZW-PA nanoparticles (almost all cells are stained red). ZW-PA exhibited much better anti-biofilm ability than the same concentration of ampicillin.

For the mature biofilm pre-fabricated for 3 days (1 + 3 days), ZW-PA nanoparticles could still demonstrate excellent anti-biofilm activity. It can be seen in Figure 2, plot (o) that most of the cells in the biofilm were killed (they are stained red). Figure 2, plot (p) shows that the density and height of the biofilm were significantly decreased.

For the biofilm pre-cultivated for 5 days (1 + 5 days) with higher cell density and height, ZW-PA nanoparticles could still efficiently kill the cells in the biofilm matrix and reduce the cell density (Figure 2, plot (q)). However, ZW-PA could not entirely destroy the whole biofilm, as there were still 15 µm of biofilm remaining (see Figure 2, plot (r)). Nevertheless, compared with the sample treated with ampicillin in which the height was slightly reduced from 25 µm (Figure 2, plot (f)) to 24 µm (Figure 2, plot (I)), ZW-PA nanoparticles revealed much better anti-biofilm ability than ampicillin. These results clearly indicate that ZW-PA nanoparticles is a highly efficient anti-biofilm agent.

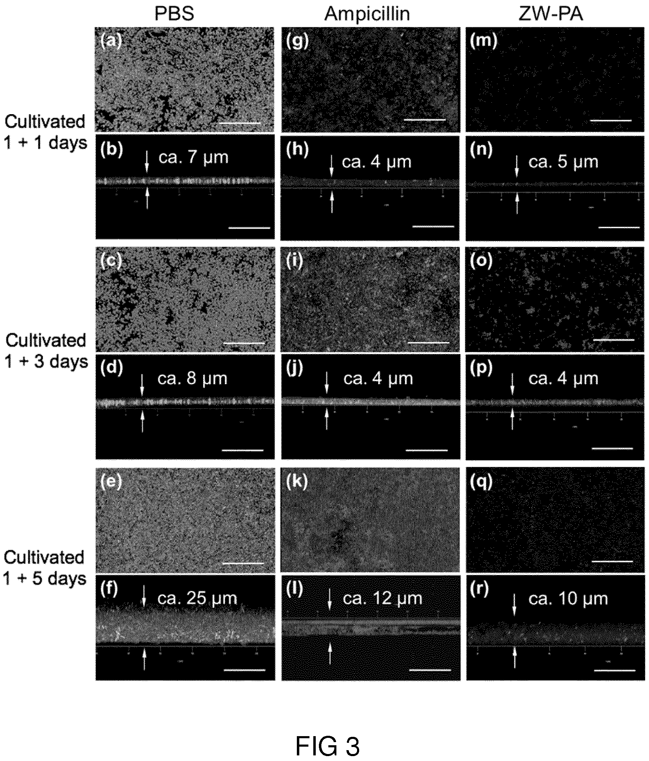

Biofilms are considered to be highly resistant to antibacterial agents due to restricted penetration of antibacterial agents into biofilms, slow growth owing to nutrient limitation, expression of genes involved in the general stress response, and emergence of a biofilm-specific phenotype. Thus, it is important to investigate the emergence of resistances against a new antibacterial agent used for combating biofilms. Therefore, ZW-PA nanoparticles in a concentration of 200 nM were applied to a biofilm pre-fabricated for 1 day. Subsequently, the biofilm was incubated with ZW-PA nanoparticles and investigated after 1 day incubation (24 h, cultivated 1 + 1 days), after 3 days incubation (72 h, cultivated 1 + 3 days), and after 5 days incubation (120 h, cultivated 1 + 5 days). PBS and 200 nM ampicillin were used as controls. The corresponding results are shown in Figure 3, plots (a)-(l). The scale bars in all plots of Figure 3 represent 50 µm.

Upon incubation in PBS, the biofilm grew from the 2^{nd} (1^{st} + 1 day) to the 6^{th} (1^{st} + 5 days) day from 7µm height to 25 mm height. Only green (living) cells could be observed (Figure 3, plots (a)-(f)).

After the biofilm was treated with ampicillin for 24 h, a part of the cells could be killed (more red than green cells are visible in Figure 3, plot (g)) and the cell density of the biofilm was reduced (Figure 3, plot (g)). However, when the ampicillin-treated biofilm was further incubated, the biofilm could gradually reform, accompanied with an increasing cell density and height. In Figure 3, plot (i) more green than red cells are visible, whereas in Figure 3, plot (k) more red than green cells can be seen. These phenomena were coincident with the results reported in the literature¹⁶ indicating that ampicillin does not kill all the cells, but that a small fraction of resistant cells emerged in the deeper layers of the biofilm. This effect is responsible for the regrowth of the biofilm.

In case of biofilms treated with ZW-PA nanoparticles, different results could be observed (Figure 3, plots (m)-(r)). It could be seen that the confluent cells in the biofilm matrix were killed into sparse dead cell colonies that were all stained red in Figure 3, plot (m)). The cell density did not increase even after the biofilm was further cultivated (Figure 3, plot (o) and plot (q)).

Besides, from the side views shown in Figure 3, plots (n), (p) and (r), the cells from top to bottom in the biofilm matrix were almost dead (they were stained red), and the regrowth rate of the biofilm was much slower compared with that incubated with ampicillin.

These results indicate that the antibacterial activity of ZW-PA nanoparticles was available over 6 days (1 + 5 days). Apparently, it is not so easy for the cells in the biofilm matrix to generate a resistance against ZW-PA nanoparticles. This further underlines the superior properties of ZW-PA as anti-biofilm agent.

### List of references cited in the preceding sections

- 1: Global Risks 2013 (Ed.: L. Howell), World Economic Forum, Geneva, 2013, pp. 28-35.
- 2: Bush, K.; Courvalin, P.; Dantas, G.; Davies, J.; Eisenstein, B.; Huovinen, P.; Jacoby, G. A.; Kishony, R.; Kreiswirth, B. N.; Kutter, E.; Lerner, S. A.; Levy, S.; Lewis, K.; Lomovskaya, O.; Miller, J. H.; Mobashery, S.; Piddock, L. J. V.; Projan, S.; Thomas, C. M.; Tomasz, A.; Tulkens, P. M.; Walsh, T. R.; Watson, J. D.; Witkowski, J.; Witte, W.; Wright, G.; Yeh, P.; Zgurskaya, H. I. Nat Rev Micro 2011, 9, 894.
- 3: Flemming, H.-C.; Wingender, J.; Szewzyk, U.; Steinberg, P.; Rice, S. A.; Kjelleberg, S. Nat Rev Micro 2016, 14, 563.
- 4a: Walsh, C. T.; Wencewicz, T. A. J Antibiot 2014, 67, 7*.*
- 4b: Ghosh, C.; Haldar, J. ChemMedChem 2015, 10, 1606.
- 5a: Dua, K.; Shukla, S. D.; Tekade, R. K.; Hansbro, P. M. Drug Deliv. and Transl. Res. 2017, 7, 179.
- 5b: Antibiotics: Targets, Mechanisms and Resistance, John Wiley & Sons, 2013, p 1.
- 6: Zasloff, M. Nature 2002, 415, 389.
- 7: Tew, G. N.; Scott, R. W.; Klein, M. L.; DeGrado, W. F. Acc. Chem. Res. 2010, 43, 30.
- 8: Seil, J. T.; Webster, T. J. Int. J. Nanomedicine 2012, 7, 2767.
- 9a: Liu, K.; Liu, Y.; Yao, Y.; Yuan, H.; Wang, S.; Wang, Z.; Zhang, X. Angew. Chem. Int. Ed. 2013, 52, 8285.
- 9b: Roy, I.; Shetty, D.; Hota, R.; Baek, K.; Kim, J.; Kim, C.; Kappert, S.; Kim, K. Angew. Chem. Int. Ed. 2015, 54, 15152.
- 10: Wang, L.-S.; Gupta, A.; Rotello, V. M. ACS Infect. Dis. 2016, 2, 3.
- 11: Webber, M. J.; Appel, E. A.; Meijer, E. W.; Langer, R. Nat Mater 2016, 15, 13.
- 12: Ogoshi, T.; Yamagishi, T.-a. In Pillararenes; The Royal Society of Chemistry: 2016, p 1.
- 13a: Jie, K.; Zhou, Y.; Yao, Y.; Huang, F. Chem. Soc. Rev. 2015, 44, 3568.
- 13b: Yu, G.; Jie, K.; Huang, F. Chem. Rev. 2015, 115, 7240. Ogoshi, T.; Yamagishi, T.-a.; Nakamoto, Y. Chem. Rev. 2016, 116, 7937.
- 14a: Joseph, R.; Naugolny, A.; Feldman, M.; Herzog, I. M.; Fridman, M.; Cohen, Y. J. Am. Chem. Soc. 2016, 138, 754*.*
- 14b: Joseph, R.; Kaizerman, D.; Herzog, I. M.; Hadar, M.; Feldman, M.; Fridman, M.; Cohen, Y. Chem. Commun. 2016, 52, 10656.
- 15a: Si, W.; Li, Z.-T.; Hou, J.-L. Angew. Chem. Int. Ed. 2014, 53, 4578.
- 15b: Zhang, M.; Zhu, P.-P.; Xin, P.; Si, W.; Li, Z.-T.; Hou, J.-L. Angew. Chem. Int. Ed. 2017, 56, 2999.
- 16: Ito, A.; Taniuchi, A.; May, T.; Kawata, K.; Okabe, S. Appl Environ Microbiol. 2009, 75, 4093.
- 17: Liu, Y.; Busscher, H. J.; Zhao, B.; Li, Y.; Zhang, Z.; van der Mei, H. C.; Ren, Y.; Shi, L. ACS Nano 2016, 10, 4779*.*

## Claims

1. Zwitterionic pillararene derivative having a structure according to general formula (I), 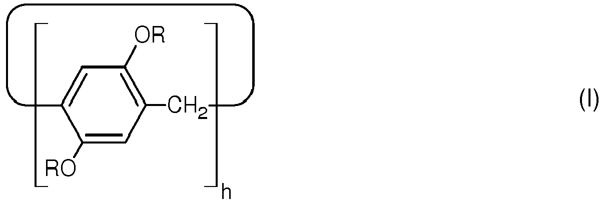 wherein
R is
D is OSO₃⁻ Y⁺, SO₃⁻ Y⁺, or CO₂⁻ Y⁺,
E is CH₃ or CH₂-CH₃,
X is Cl or Br,
Y is Na or K,
Z is N or P,
h is 5, 6, 7, 8, 9, or 10,
m is 1, 2, 3, 4, 5, 6, 7, 8, or 9, and
n is 1, 2, 3, 4, 5, 6, 7, 8, or 9.

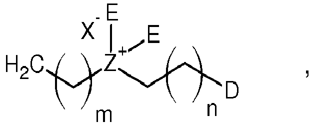

2. Zwitterionic pillararene derivative according to claim 1, **characterized in that** h is 5 or 6.

3. Zwitterionic pillararene derivative according to claim 1 or 2, **characterized in that** E is CH₂-CH₃.

4. Zwitterionic pillararene derivative according to any of the preceding claims, **characterized in that** m is 1 and n is 5.

5. Zwitterionic pillararene derivative according to any of the preceding claims, **characterized in that** D is OSO₃⁻ Y⁺.

6. Nanoparticle comprising a plurality of zwitterionic pillararene derivative molecules according to any of the preceding claims.

7. Use of a zwitterionic pillararene derivative according to any of claims 1 to 5 or of a nanoparticle according to claim 6 as anti-biofilm compound in vitro and/or as antibacterial compound in vitro.

8. Use according to claim 7, **characterized in that** the anti-biofilm compound is used for providing a substrate with biofilm-inhibiting properties.

9. Zwitterionic pillararene derivative according to any of claims 1 to 5 or nanoparticle according to claim 6 for use as medicament.

10. Zwitterionic pillararene derivative according to any of claims 1 to 5 or nanoparticle according to claim 6 for use according to claim 9, **characterized in that** the medicament is an anti-biofilm medicament and/or an antibacterial medicament.

11. Article, comprising a matrix having a) a matrix material and b) a pillararene derivative according to any of claims 1 to 5 or a nanoparticle according to claim 6.

12. Article according to claim 11, **characterized in that** the pillararene derivative or the nanoparticle is embedded into the matrix material.

13. Article according to claim 11 or 12, **characterized in that** the pillararene derivative or the nanoparticle is coated onto a surface of the matrix material.

14. Article according to any of claims 11 to 13, **characterized in that** article is intended to be in contact with a human or animal body or a human or animal body fluid for a period of time lasting at least 6 hours.

15. Method for manufacturing a zwitterionic pillararene derivative according to any of claims 1 to 5, comprising the following steps:
reacting a compound of general formula (II) 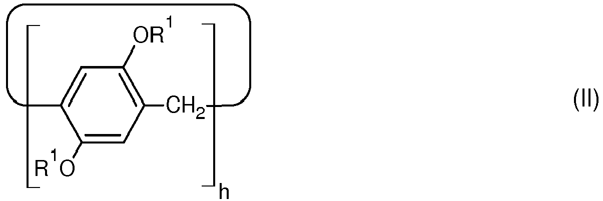
wherein
R1 is
E is CH₃ or CH₂-CH₃,
Z is N or P,
h is 5, 6, 7, 8, 9, or 10, and
m is 1, 2, 3, 4, 5, 6, 7, 8, or 9, 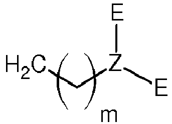
with a compound of general formula (III) 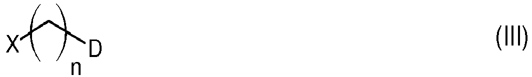 wherein
D is OSO₃⁻ Y⁺, SO₃⁻ Y⁺, or CO₂⁻ Y⁺,
X is Cl or Br,
Y is Na or K, and
n is 1, 2, 3, 4, 5, 6, 7, 8, or 9,
to obtain a zwitterionic pillararene derivative having a structure as defined in any of claims 1 to 5.
